# EUROPEAN PATENT APPLICATION

(11) **EP 4 790 131 A1**
(43) Date of publication of application: **12.08.2026**
(21) Application number: 25157141.0
(22) Date of filing: 11.02.2025
(51) Int. Cl.: C12N 1/12, B01D 53/85, C01F 11/02, C01F 11/18, C04B 2/00

(54) **PROCESS FOR PRODUCING CALCIUM HYDROXIDE**

(71) Applicant: SkyCell AG, 6300 Zug (CH)
(72) Inventor: ROS, Nico, 4125 Riehen (CH); HEGGLIN, Michael, Boston, MA 02120 (US); VOLSA, Keitlina, 8105 Regensdorf (CH); BECK, Gesa, 13591 Berlin (DE)
(74) Representative: SONN Patentanwälte GmbH & Co KG

(57) **Abstract**

A process for producing calcium hydroxide, comprising:
reacting calcium carbonate with an acid in an aqueous solution at a temperature of 10-50°C to produce a calcium ion solution and carbon dioxide,
separating the carbon dioxide from the calcium ion solution,
treating the calcium ion solution to form calcium hydroxide,
directing the separated carbon dioxide to a photobioreactor containing microalgae, and
growing the microalgae in the photobioreactor using the carbon dioxide through photosynthesis.

## Description

The invention refers to a process for producing calcium hydroxide from a source of calcium carbonate.

Lime (CaO) is conventionally manufactured in a highly carbon intensive process via the calcination of limestone (CaCO₃) in a kiln. The process is most often fuelled by fossil fuels such as coal, petroleum coke, oil and gas. The combustion of these fuels contributes around one-third of total carbon dioxide (CO₂) emissions associated with CaO manufacturing, with process emissions contributing the other two-thirds. All in all, for every tonne of CaO produced, around one tonne of CO₂ is emitted.

The lime industry, like many other manufacturing industries, has ambitious plans to meet net-zero by 2050 in alignment with the environmental objectives set by the Paris Climate Agreement. Various technologies are being developed in order to meet these targets, such as hydrogen fuels, electrified kilns, and carbon capture and utilisation utilising adsorbent materials. These technologies are high in energy demand, cost (nearly double that compared to conventional lime production) and rely heavily on policy and other developments (for example a decarbonised electricity network, safe carbon transportation and storage facilities). Additionally, many technical challenges remain, and as a result, these technologies are significantly hindered in their deployment in industry.

Other alternatives such as efficiency improvements in kiln technologies and fuels (for example oxy-fuels), as well as fuel switching from fossil fuels to waste derived alternatives provide only minor improvements and are not enough to drive the necessary developments to meet net-zero.

All current options come with inherent costs that make it difficult for lime producers to compete in an international market. In this case, the lime industry cannot simply pass on the higher operating costs to customers, as they would simply source lower cost lime imported from abroad, or use alternative materials.

Accordingly, there is a need for innovative methods to produce lime at a non, or only slightly elevated temperatures, with the benefit of eliminating combustion related carbon emissions. Such methods must also encompass a low cost carbon capture approach, thereby controlling process related carbon emissions, and thus, directly contributing to the net-zero targets of the lime industry. Finally, producing lime at a non or only slightly elevated temperature will also contribute to reduced operating costs, furthering the cost competitiveness of the product.

Several processes have already been described, related to the production of metal hydroxides, via non or only slightly elevated temperature processes.

CA 3235811 A1 describes a method for producing hydroxide solids from solid substrates, such as industrial waste or rocks. The method involves subjecting a solvent and solid substrate to leach a metal cation into the solvent. The metal cation is separated from the solvent via a cathode, thereby electrolytically precipitating the metal hydroxide from the solution. In some embodiments, the chemical stimulus is an acid, for example HCl or HClO₄, or a combination thereof.

WO 2019/036676 A1 outlines a low energy route for the production of hydrated calcium and magnesium salts from alkaline industrial wastes. This involves extracting divalent ions from solids by leaching, concentrating the solution to form a concentrated divalent ion-containing solution via capacitive concentration or membrane filtration and then precipitating Ca(OH)₂ by exploiting the temperature-dependent solubility of the salts.

Although both publications provide methods for producing Ca(OH)₂ via non-elevated temperature methods, neither mention the treatment of gaseous wastes, especially carbon dioxide, liberated during leaching from solid substrates. Therefore, the environmental benefits are limited only to the elimination of emissions associated with the high temperature calcination, despite process emissions contributing two-thirds to the CO₂ footprint of lime production. Additionally, the use of chlorides makes the process unsuitable for use in the construction industry.

Some work has been conducted within the sector of algae as a carbon capture technology and use of algae derived products. For example, EP 2 5566 881 A1 relates to the field of culturing algae and use of algae derived products. This publication outlines an approach to grow algae with the help of an absorption liquid comprising carbon dioxide, alongside improving algae growth through the use or absorbent. Here, CO₂ for the process is sourced from flue gas and biogas.

It is an object of the invention to provide an environmentally friendly and economically viable process for producing calcium hydroxide that eliminates both combustion-related and process-related carbon dioxide emissions. Specifically, the invention aims to solve the technical problem of high-temperature calcination and its associated carbon emissions by providing a process that operates at non-elevated or only slightly elevated temperatures, while simultaneously addressing the challenge of process-related carbon dioxide emissions. A further object of the invention is to provide a process that produces calcium hydroxide suitable for construction applications without the use of chloride-containing acids.

To solve these objects, the invention provides a process for producing calcium hydroxide, comprising:
reacting calcium carbonate with an acid in an aqueous solution at a temperature of 10-50°C to produce a calcium ion solution and carbon dioxide,
separating the carbon dioxide from the calcium ion solution,
treating the calcium ion solution to form calcium hydroxide,
directing the separated carbon dioxide to a photobioreactor containing microalgae, and
growing the microalgae in the photobioreactor using the carbon dioxide through photosynthesis.

Thus, the invention is based on the idea of combining a low-temperature process for calcium hydroxide production with an integrated biochemical carbon capture system, where the process-generated carbon dioxide is directly utilized for microalgae growth through photosynthesis, thereby eliminating both combustion-related and process-related carbon emissions while maintaining economic viability through reduced energy consumption and the generation of valuable biomass as a by-product.

The process operates at moderate temperatures between 10-50°C, representing a departure from conventional calcination methods that require temperatures exceeding 900°C. This temperature reduction eliminates the need for fossil fuel combustion and its associated carbon dioxide emissions, which typically account for one-third of the total emissions in traditional lime production.

The initial reaction step involves treating calcium carbonate with an acid in an aqueous solution under moderate temperature conditions. This chemical decomposition produces a calcium ion solution while simultaneously releasing carbon dioxide. When calcium carbonate reacts with an acid, a neutralization reaction occurs. The calcium carbonate acts as a base and reacts with the acid to form water, carbon dioxide, and a calcium salt. For example, when calcium carbonate reacts with hydrochloric acid, the products are water, carbon dioxide, and calcium chloride.

The selection of the acid depends on the intended application of the final calcium hydroxide product. For construction industry applications, suitable acids include carbonic acid and sulfuric acid, while applications such as direct air capture allow for a broader range of acids including acetic acid, hydrochloric acid (HCl), and sulfuric acid (H₂SO₄). The chemical decomposition follows a specific reaction mechanism where calcium carbonate (CaCO₃) reacts with hydronium ions (H₃O⁺) from the acid to produce calcium ions (Ca²⁺), carbon dioxide (CO₂), and water (H₂O). This reaction can be represented by the following equation:

CaCO₃₍ₛ₎ + 2H3O⁺ → Ca²⁺ + CO₂ + 3H₂O

The reaction kinetics and efficiency are controlled through careful moderation of two parameters: the pH of the solution and the concentration of carbon dioxide. When carbonic acid is used as the reactant, the process involves the formation of intermediate ionic species. Specifically, the calcium carbonate forms a solution containing free calcium ions (Ca²⁺), hydrogen carbonate ions (HCO₃⁻), and carbonate ions (CO₃²⁻). Due to the inherent instability of both hydrogen carbonate and carbonate ions in water, these species subsequently decompose to form carbon dioxide.

Following the initial reaction, the process separates the carbon dioxide from the calcium ion solution. This separation step enables independent processing of both products - allowing the calcium ions to be converted to calcium hydroxide while directing the carbon dioxide to biological capture. The separation prevents unwanted reactions and ensures optimal efficiency in subsequent steps.

The treatment of the calcium ion solution to form calcium hydroxide can be accomplished through various methods, including base addition or controlled heating, resulting in the precipitation of the desired calcium hydroxide product.

The algae growth phase of the process begins with the separation of gaseous carbon dioxide from the aqueous reaction products (calcium ions and water). This separated carbon dioxide undergoes biochemical treatment through algae-mediated carbon fixation. The fundamental chemical process occurring in this step is photosynthesis, represented by the following equation:

6CO₂ + 6H₂O → C₆H₁₂O₆ + 6O₂

Preferably, the process employs specific algae species that have been selected for their high efficiency in carbon dioxide uptake. These species include Botryococcus braunii, Chlorella vulgaris, Chlorella kessleri, Chlorocuccum littorale, Scenedesmus sp., Chlamydomonas reinhardtii, Spirulina sp., Scenedesmus obliquus, and Nannochloropsis oculate. Each of these species has demonstrated superior capacity for carbon dioxide absorption and conversion through photosynthesis.

The carbon capture and algae growth process takes place within a photobioreactor system that can be configured for either outdoor or indoor operation. The photobioreactor's primary energy source is natural sunlight, optimizing the energy efficiency of the process. However, the system may incorporate supplementary lighting options for conditions where natural sunlight is insufficient to maintain optimal algae growth rates. These supplementary light sources may preferably be powered by renewable energy systems to maintain the process's environmental benefits.

The photobioreactor's efficiency may further be enhanced through the implementation of light management systems. These include reflective surfaces and light guides that optimize light distribution throughout the bioreactor volume.

In an advantageous embodiment of the invention, the microalgae are harvested at an optimized point in their growth cycle - specifically after the exponential growth phase but before entering the stationary phase. During the exponential growth phase, the microalgae exhibit their highest metabolic activity and carbon dioxide uptake rates, resulting in maximum biomass production and carbon fixation efficiency. By harvesting before the stationary phase, the process avoids the decreased efficiency that occurs when algal growth slows and carbon dioxide uptake rates decline. This timing also prevents the accumulation of metabolic waste products that typically occurs during the stationary phase, which could otherwise inhibit further growth and reduce the system's carbon capture efficiency. Furthermore, harvesting at this point ensures that the photobioreactor maintains its maximum operational efficiency by preventing the development of density-related light limitation issues that can occur if the culture becomes too dense.

In a preferred embodiment of the invention, the calcium ion solution is treated by adding a base containing hydroxide ions, followed by a filtration step to isolate the precipitated calcium hydroxide. The addition of hydroxide ions from bases such as sodium hydroxide, potassium hydroxide, or ammonium hydroxide triggers an immediate precipitation reaction with the calcium ions in solution, forming solid calcium hydroxide through a controlled and predictable chemical process. The subsequent filtration step effectively separates the solid calcium hydroxide from the reaction solution, enabling the recovery of a pure product while simultaneously allowing for the potential recycling of the remaining solution components.

In a particularly advantageous embodiment of the invention, the calcium ion solution is subjected to controlled heating to form calcium hydroxide through a two-stage thermal process. The controlled application of heat first promotes the precipitation of calcium hydroxide and subsequently drives the evaporation of water to obtain a solid product. This thermal treatment, operating below the decomposition temperature of calcium hydroxide (400°C), achieves both conversion and drying in a single step while eliminating the need for additional chemical reagents or separate drying steps.

In a further preferred embodiment of the invention, the filtered or precipitated calcium hydroxide undergoes a controlled drying process at temperatures below its decomposition temperature in a carbon dioxide free environment to obtain a dry powder product. The operation below the decomposition temperature ensures preservation of the chemical integrity of the calcium hydroxide while achieving effective moisture removal, while the carbon dioxide free environment prevents unwanted carbonation reactions that would convert the calcium hydroxide back to calcium carbonate. These controlled conditions result in a high-purity dry powder form of calcium hydroxide that maintains its full reactivity, making it particularly suitable for both construction applications and use as an adsorbent in direct air carbon capture systems.

In a preferred embodiment, the method further comprises maintaining a carbon dioxide concentration in the photobioreactor between 3% and 20%. This controlled condition may preferably be achieved through a supply of concentrated carbon dioxide, managed via a buffer tank positioned between the calcium hydroxide production and the photobioreactor. The buffer tank serves to maintain stable carbon dioxide concentrations in the growth media, with the specific concentration within the 3-20% range being selected based on the requirements of the particular algae species being cultivated. This control of carbon dioxide concentration ensures optimal conditions for photosynthesis while preventing inhibitory effects from concentration fluctuations, thereby maximizing the efficiency of the carbon capture process.

In another preferred embodiment, the method further comprises maintaining a pH level in the photobioreactor between 6.5 and 8.5. This pH range represents optimal conditions for both algal metabolism and carbon dioxide dissolution in the growth media. The controlled pH environment helps optimize the availability of dissolved carbon dioxide for the microalgae while preventing pH-related stress that could impair cellular function or growth rates.

In an advantageous embodiment, the method further comprises introducing the carbon dioxide to the photobioreactor using an adsorbent material for controlled release of the carbon dioxide to the microalgae. This addresses the carbon dioxide's low solubility in aqueous media, which can lead to inhibited algae growth and system inefficiencies. The adsorbent material acts as a temporary storage medium, holding the carbon dioxide and releasing it gradually to the algae culture. This controlled release mechanism ensures a steady supply of carbon dioxide to the microalgae, optimizing its availability for photosynthesis while preventing localized high concentrations that could inhibit growth.

In another preferred embodiment, the invention addresses the carbon dioxide's low solubility in aqueous media through a hydrogen carbonate-based delivery system enhanced with specific enzymes. By utilizing hydrogen carbonate, which exhibits higher solubility in water compared to carbon dioxide, the system provides a more stable and readily available form of carbon for the microalgae. Here, the method may further comprise adding enzymes to facilitate carbon dioxide uptake by the microalgae, such as carbonic anhydrase enzyme (CAE) and RuBisCo (Ribulose-1,5-bisphosphate carboxylase/oxygenase). Carbonic anhydrase enzyme (CAE) catalyzes the reversible hydration of carbon dioxide to form carbonic acid, which dissociates to hydrogen carbonate and protons, thereby maintaining an optimal equilibrium between carbon dioxide and hydrogen carbonate forms. Further, RuBisCo (Ribulose-1,5-bisphosphate carboxylase/oxygenase) catalyzes the incorporation of carbon dioxide into organic compounds during photosynthesis, enabling efficient conversion of the captured carbon into algal biomass.

These two approaches, i.e. using an adsorbent material and hydrogen carbonate in combination with adding enzymes, can be implemented independently or in combination.

In some embodiments, the carbon dioxide may also be directly bubbled into the algae culture medium to provide aeration for the algae.

According to a preferred embodiment of the invention, the calcium carbonate used as starting material may be sourced from at least one of primary materials, industrial waste, and construction and demolition waste. The ability to utilize calcium carbonate from industrial waste or construction and demolition waste enables the process to function as part of a circular economy, reducing waste and the need for primary resource extraction. When using primary materials, such as limestone, the process can be optimized for consistent feed composition, while the capability to process waste materials allows for the remediation of industrial byproducts and construction waste that might otherwise be landfilled.

In another preferred embodiment, the method further comprises grinding or pulverizing the calcium carbonate into particles prior to the reaction with the acid. By reducing the calcium carbonate to smaller particles, the method increases the surface area available for reaction with the acid, thereby improving reaction kinetics and conversion efficiency. The increased surface area-to-volume ratio of the pulverized material enables more efficient acid-base interactions, leading to faster and more complete decomposition of the calcium carbonate during the subsequent reaction step.

The invention will now be described with reference to an exemplary embodiment. The following example case is provided to demonstrate the overall concept of a non or only slightly elevated temperature process for producing Ca(OH)₂ alongside biochemical carbon capture and utilization.

In this example, a lime production facility with a capacity of 1,000 tonnes of Ca(OH)₂ per year requires input of CaCO₃ at a rate of 100 - 200 kg/hr, assuming 24 hours operation, 365 days a year. This process produces between 300 - 400 tonnes of algae per year, with the capacity of absorbing all process CO₂ emissions. To maintain an optimal CO₂ ratio within the growth media, a CO₂ buffer tank of between 1500 - 2000 L is sufficient. Such a set up requires a photobioreactor size of around 400 - 500 kL.

Given that this is a non or only slightly elevated temperature process, there are minimal energy requirements for the production of the Ca(OH)₂. The highest energy demands come from the energy required by the microalgae for photosynthesis, which is between 200 - 8000 kWh per day, depending on growth stage of the microalgae. This energy can be provided by natural sunlight. If needed, the efficiency of the light source is improved via strategically selected and placed reflective surfaces and light guides to direct and distribute light more effectively within the phytobioreactor. When required, the photobioreactor is supplemented with artificial lighting powered by renewable energy systems.

The lime produced absorbs around 600 tonnes of atmospheric CO₂ during its application.

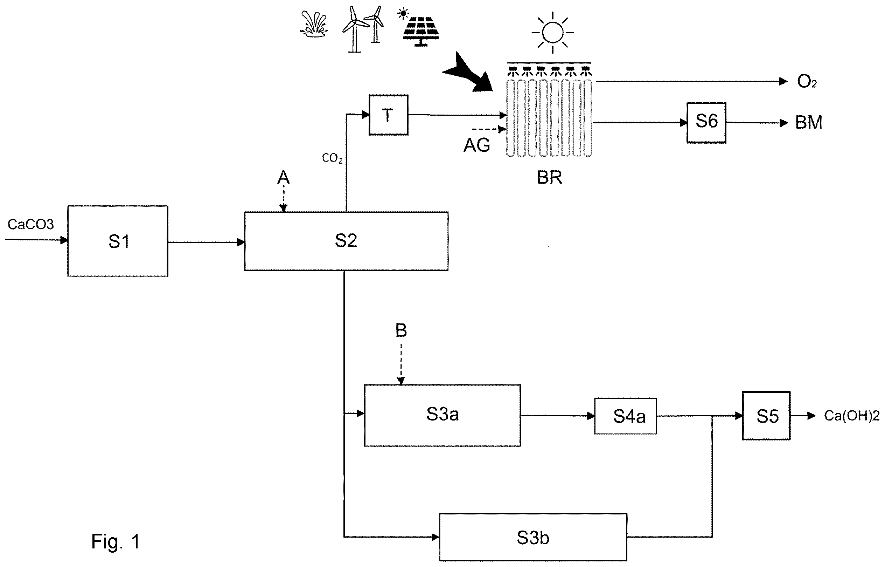

Further, the process according to the invention is illustrated in Figure 1 and comprises the following steps:
In a first step (S1), calcium carbonate undergoes an optional preprocessing operation of grinding and pulverizing to achieve appropriate particle size. The preprocessed calcium carbonate is then subjected to chemical decomposition (S2) through reaction with an acid (A) at room temperature, according to the reaction: CaCO₃ + 2H₃O⁺ → Ca²⁺ + CO₂ + 3H₂O, thereby producing a calcium ion (Ca²⁺) solution and carbon dioxide (CO₂).

The process then divides into two parallel pathways for treating the respective products:
The calcium ion solution is processed via one of two alternative routes. In a first route, the calcium ion solution undergoes a step (S3a) of base (B) addition at room temperature to precipitate calcium hydroxide according to the reaction: Ca²⁺ + 2OH⁻→ Ca(OH)₂, followed by a filtration step (S4a) to isolate the precipitated Ca(OH)₂. In a second route, the calcium ion solution undergoes direct heating (S3b) at temperatures between 50-100°C to form Ca(OH)₂.

The calcium hydroxide obtained from either route is subjected to a drying operation (S5) at temperatures between 50-200°C in a CO₂-free environment to produce dry calcium hydroxide as the final product.

Simultaneously, the carbon dioxide is processed through a biochemical carbon capture pathway comprising directing the CO₂ to a buffer tank (T) for controlled delivery to a photobioreactor system (BR), wherein microalgae convert the CO₂ through photosynthesis according to the reaction: 6CO₂ + 6H₂O → C₆H₁₂O₆ + 6O₂, producing oxygen as a by-product and algae biomass (BM). The algae biomass subsequently undergoes a drying operation (S6) at temperatures between 40-70°C.

The process is powered by renewable energy sources throughout all steps.

## Claims

**1.** A process for producing calcium hydroxide, comprising:
reacting calcium carbonate with an acid in an aqueous solution at a temperature of 10-50°C to produce a calcium ion solution and carbon dioxide,
separating the carbon dioxide from the calcium ion solution,
treating the calcium ion solution to form calcium hydroxide,
directing the separated carbon dioxide to a photobioreactor containing microalgae, and
growing the microalgae in the photobioreactor using the carbon dioxide through photosynthesis.

**2.** The process according to claim 1, further comprising harvesting the microalgae after an exponential growth phase and before a stationary phase.

**3.** The process according to claim 1 or 2, wherein treating the calcium ion solution comprises adding a base containing hydroxide ions to precipitate calcium hydroxide, followed by filtration of calcium hydroxide.

**4.** The process according to claim 3, wherein the base is selected from the group consisting of sodium hydroxide, potassium hydroxide, and ammonium hydroxide.

**5.** The process according to claim 1 or 2, wherein treating the calcium ions comprises heating the calcium ion solution.

**6.** The process according to any one of claims 1 to 5, further comprising drying the calcium hydroxide at a temperature below 400°C in a carbon dioxide free environment in order to obtain calcium hydroxide as a dry powder.

**7.** The process according to any one of claims 1 to 6, wherein the acid is selected from the group consisting of carbonic acid, sulfuric acid, acetic acid, and hydrochloric acid.

**8.** The process according to any one of claims 1 to 7, wherein the microalgae is selected from the group consisting of Botryococcus braunii, Chlorella vulgaris, Chlorella kessleri, Chlorocuccum littorale, Scenedesmus sp., Chlamydomonas reinhardtii, Spirulina sp., Scenedesmus obliquus, and Nannochloropsis oculate.

**9.** The process according to any one of claims 1 to 8, further comprising maintaining a carbon dioxide concentration in the photobioreactor between 3% and 20%.

**10.** The process according to any one of claims 1 to 9, further comprising maintaining a pH level in the photobioreactor between 6.5 and 8.5.

**11.** The process according to any one of claims 1 to 10, wherein the photobioreactor is provided with natural sunlight and optionally supplementary artificial lighting powered by renewable energy.

**11.** The process according to any one of claims 1 to 10, further comprising controlling the introduction of carbon dioxide to the photobioreactor using a buffer tank positioned between the calcium hydroxide production and the photobioreactor.

**12.** The process according to any one of claims 1 to 11, wherein introducing the carbon dioxide to the photobioreactor comprises using an adsorbent material for controlled release of the carbon dioxide to the microalgae.

**13.** The process according to any one of claims 1 to 12, further comprising adding enzymes to facilitate carbon dioxide uptake by the microalgae.

**14.** The process according to claim 13, wherein the enzymes comprise at least one of carbonic anhydrase enzyme (CAE) and Ribulose-1,5-bisphosphate carboxylase/oxygenase (RuBisCo).

**15.** The process according to any one of claims 1 to 14, wherein the calcium carbonate is sourced from at least one of primary materials, industrial waste, and construction and demolition waste.

**16.** The process according to any one of claims 1 to 15, further comprising grinding or pulverizing the calcium carbonate into particles prior to the reaction with the acid.
